(19) Europäisches Patentamt
European Patent Office
Office européen des brevets

(11) EP 2 654 739 B1

(12) # EUROPEAN PATENT SPECIFICATION

(45) Date of publication and mention of the grant of the patent:
**22.07.2020 Bulletin 2020/30**

(21) Application number: **11851699.6**

(22) Date of filing: **22.12.2011**

(51) Int Cl.:
***A61K 9/70*** (2006.01) ***A61K 31/27*** (2006.01)
***A61P 25/28*** (2006.01)

(86) International application number:
**PCT/KR2011/009994**

(87) International publication number:
**WO 2012/087047 (28.06.2012 Gazette 2012/26)**

(54) **PERCUTANEOUS ABSORPTION PREPARATION CONTAINING RIVASTIGMINE**

RIVASTIGMIN ENTHALTENDES PRÄPARAT FÜR PERKUTANE RESORPTION

PRÉPARATION POUR ABSORPTION PERCUTANÉE CONTENANT DE LA RIVASTIGMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2010 KR 20100134692**

(43) Date of publication of application:
**30.10.2013 Bulletin 2013/44**

(73) Proprietor: **Samyang Biopharmaceuticals Corporation**
**Seoul 110-725 (KR)**

(72) Inventors:
- **YU, Hyun-Suk**
  **Gunpo-si**
  **Gyeonggi-do 435-745 (KR)**
- **LEE, Young-Moo**
  **Seoul 138-790 (KR)**
- **KIM, Hyun-Woo**
  **Yongin-si**
  **Gyeonggi-do 448-110 (KR)**
- **KIM, Hee-Sook**
  **Asan-si**
  **Chungcheongnam-do 336-722 (KR)**

(74) Representative: **Brann AB**
**P.O. Box 3690**
**Drottninggatan 27**
**103 59 Stockholm (SE)**

(56) References cited:

| | |
|---|---|
| **WO-A1-99/34782** | **WO-A1-2007/064407** |
| **WO-A1-2007/064407** | **JP-A- 2009 022 730** |
| **KR-A- 20100 080 681** | **KR-A- 20100 080 681** |
| **US-A1- 2004 258 741** | **US-A1- 2004 258 741** |
| **US-A1- 2005 129 748** | **US-A1- 2008 044 461** |

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 654 739 B1

## Description

### Field of the Invention

The present invention relates to a percutaneous absorption preparation containing rivastigmine as an active ingredient.

### Background Art

With a steady increase in the average human life expectancy thanks to advances in medicine and dietary pattern, our world is undergoing a rapid change into aging society. This global trend toward population aging is greatly increasing the number of old people in need of long-term care services. Senile dementia, one of the most devastating diseases associated with aging, is the most common disease of the elderly in the world and known as a degenerative disorder that destroys the individual, more often than not, the lives of the individual's family to cause social problems.

Senile dementia is classified into two major types: Alzheimer's type and vascular dementia. The most common form of senile dementia, Alzheimer's disease, is a central nervous system disorder that begins with memory disorder, develops conduct disorder, language problems, and poor judgment, and ends up with devastation of personality.

There have been many studies on dementia, but the exact reason of development of dementia is not yet known. Due to different etiogical and pathophysiological factors of dementia, there exists no remedy administered alone to treat dementia. In an attempt to derive an approach to treatment of the traditional degenerative dementia, studies have been made on the use of inhibitors for inhibiting cholinesterase (ChE) that is an enzyme for hydrolyzing a neurotransmitter, acetylcholine, Cholinesterase. There are two types of cholinesterase: acetylcholinesterase (AChE) and butylcholinesterase (BuChE). Acetylcholinesterase, which is an enzyme that hydrolyzes one of the neurotransmitters mediating the activity of parasympathetic nerve, acetylcholine, into choline and acetate, is released from endoplasmic reticulum membranes and anchors onto cell membranes to conduct its functions. This enzyme is mostly present in cholinergic nerves and their peripherals, especially in neuromuscular junctions, and also found in blood plasma, liver, or other tissues.

Hence the existing cures for Alzheimer's type dementia are mostly acetylcholinesterase inhibitors, including, for example, donepezil (Aricept®), tacrine (Cognex®), galantamine (Razadyne®), or the like. Among these, rivastigmine is an inhibitor acting on both acetylcholinesterase and butylcholinesterase and used as an active ingredient in a percutaneous absorption preparation commercially available as Exelon® Patch (Novartis Korea). Exelon® Patch commercially available comes in two sizes, 10 $cm^2$ (content of active ingredient: 18 mg) and 5 $cm^2$ (content of active ingredient: 9 mg).

Rivastigmine, which has amine and carbonyl functional groups, can cause skin irritation when used as an ingredient of percutaneous absorption preparations, and the functional groups are ready to participate in interactions with an adhesive used in the percutaneous absorption preparation to inhibit diffusion and permeation of rivastigmine into the body through the skin. Exelon® Patch, which is a percutaneous absorption preparation using rivastigmine as an active ingredient, causes side effects on the skin, such as erythema, edema, or the like (See. Clinical Drug Investigation, Jan. 1, 2010, Vol 30, pp 41 - 49), and most of those side effects on the skin are reportedly caused by the drug.

Accordingly, there is a need for developing a percutaneous absorption preparation that uses less rivastigmine than conventional rivastigmine patches to reduce skin irritation, exhibits a high permeability into the skin, carrying a sufficient amount of the drug necessary for treatment of dementia, has adhesiveness strong enough to stick to the skin, and is easy to manufacture.

As a prior art to solve the problems, WO 99/34782 provides a technique to stabilize a pharmaceutical composition containing rivastigmine by using an antioxidant such as tocopherol acetate, ascorbyl palmitate, or ascorbic acid. The document also provides a percutaneous absorption system comprising (a) a backing layer acting as a support; (b) a layer containing a pharmaceutical composition comprising rivastigmine and an antioxidant; (c) an independent adhesive layer for adhesion to the patient's skin to make the pharmaceutical composition released into the skin; and (d) a release liner.

The percutaneous absorption system of the prior art, however, necessarily uses an antioxidant to stabilize rivastigmine and has a complex four-layered structure including an independent adhesive layer, which brings about difficulty of manufacture.

WO 07/064407 provides a technique of considerably improving the adhesive characteristic of a rivastigmine-containing percutaneous absorption preparation without any change in the release profile of rivastigmine by independently forming an adhesive layer containing a silicone polymer and an adhesive in the preparation. More specifically, the document of the prior art provides a transdermal therapeutic system comprising (a) a backing layer acting as a support; (b) a reservoir layer containing rivastigmine and one or more polymers; and (c) an adhesive layer containing a silicone polymer and an adhesive.

This prior art, which provides properties not sufficient to stick the preparation to the skin with the drug-containing layer alone in the same manner as WO 99/34782, is constructed to have an additional adhesive layer not affecting the

drug delivery into the skin, resulting in inconvenience of separately forming the additional adhesive layer and hence an additional production cost.

European Patent EP 1437130 provides a percutaneous absorption preparation for the treatment of dementia containing, as an active ingredient, donepezil, zanapezil, icopezil, or ER-127528 and having a skin permeation rate of 1.2 μg/cm$^2$/h or above. More specifically, the document of the prior art provides a percutaneous absorption preparation comprising (a) a backing; (b) a hydrophobic matrix layer containing, as a dispersed active ingredient, donepezil, zanapezil, icopezil, or ER-127528 and (c) a liner, and having a skin permeation rate of at least 1.2 μg/cm$^2$/h.

The document focuses on a selective acetylcholinesterase such as donepezil, zanapezil, icopezil, or ER-127528, which are different in mechanism from rivastigmine acting on both acetylcholinesterase and butylcholinesterase. Rivastigmine (formula 1) is a substance completely different in chemical structure from the donepezil of the document. Rivastigmine (formula 1) and donepezil (formula 2) are represented as follows:

[Formula 1]

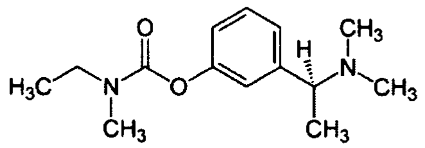

[Formula 2]

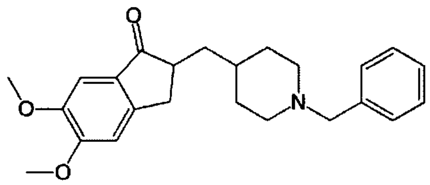

At the room temperature, donepezil is crystalline and rivastigmine is liquid. These two drugs are to be handled differently in regard to miscibility between drug and adhesive,physical properties, and skin permeation rate in preparing a percutaneous absorption preparation.

Moreover, the document specifies that the adhesive composition comprises an organic acid or pharmaceutically acceptable salts thereof, preferably acetic acid in order to acquire a skin permeation rate of 1.2 μg/cm$^2$/h or above. In the document, comparative examples 1, 2 and 3 provide percutaneous absorption preparations made in the same manner as described in examples 1, 2 and 3 excepting that sodium acetate is not used. The preparations of the comparative examples 1, 2 and 3 have a skin permeation rate of no more than 0.1 to 0.4 μg/cm$^2$/h. According to the document, the preparations not containing an organic acid salt such as acetate have a considerable deterioration in the skin permeation amount of the drug, consequently with a difficulty of acquiring an effective potency.

JP 2009/22730 describes a drug-in-adhesive patch comprising 1 wt% rivastigmine, 20 wt% styrene-isoprene-styrene, 29 wt% Arcon P-100, 8 wt% polyisobutylene, 35 wt% liquid paraffin, 3 wt% crotamiton, 2 wt% butyl hydroxytoluene and 2 wt% menthol. The amount of rivastigmine in the patch is 0.1 mg/cm$^2$.

## SUMMARY OF THE INVENTION

The present invention is to compensate for the weakness of the prior arts and to provide a percutaneous absorption preparation with a simple structure that acquires a high skin permeability of rivastigmine, reduces side effects such as skin irritation, provides good adhesion and wearing properties to the skin, and has easiness of manufacture.

The inventors of the present invention have found out that the use of a styrene-isoprene-styrene block copolymer as an adhesive base enables it to provide a rivastigmine percutaneous preparation improved in stability and potency with the most basic structure.

More specifically, according to the present invention, when using a styrene-isoprene-styrene block copolymer as an adhesive base, it is possible to provide a percutaneous absorption preparation through a simple preparation process that does not involve formation of a separate adhesive layer including a silicone adhesive or the like so that the preparation acquires a high skin permeation rate of rivastigmine (greater than 18 μg/cm$^2$/h) and a high cumulative skin permeation amount of rivastigmine (more than 30 % of the initial content in 24 hours) without incorporating an additional substance such as an organic acid or an organic acid salt, and uses only a smaller amount of rivastigmine (at least 1.0 and less than 1.8 mg/cm$^2$) to reduce skin irritation than the conventional patch preparations commercially available.

It is an object of the present invention to provide a percutaneous absorption preparation that has easiness of preparation and reduces skin irritation caused by contact of drugs on the skin by enhancing the percutaneous absorption efficiency of rivastigmine and reducing the drug dose necessary to acquire an appropriate potency.

In one aspect of the present invention to achieve the above object, there is provided a percutaneous absorption preparation that comprises (a) a backing layer; and (b) a drug-containing layer comprising rivastigmine free base or a pharmaceutically acceptable salt thereof, a styrene-isoprene-styrene block copolymer, a tackifier resin and a plasticizer wherein the percutaneous absorption preparation is free from an additional intermediate layer or an additional adhesive layer; the drug-containing layer (b) does not comprise an organic acid or a salt thereof; and the rivastigmine free base or pharmaceutically acceptable salt thereof is included in an amount of at least 1.0 $mg/cm^2$ and less than 1.8 $mg/cm^2$, and the rivastigmine free base or pharmaceutically acceptable salt thereof, the styrene-isoprene-styrene block copolymer, the tackifier resin, and the plasticizer are included at a weight ratio of 1-2 : 2-5 : 2~5 : 0.1~2.

Hereinafter, the present invention will be described in further detail.

The present invention is characterized by a rivastigmine percutaneous absorption preparation using a styrene-isoprene-styrene block copolymer as an adhesive base.

Rivastigmine, which has amine and carbonyl groups, causes skin irritation when used as a percutaneous preparation, and has these functional groups susceptible to an interaction with the functional groups of an adhesive. The interaction between the drug and the adhesive may inhibit the diffusion and permeation process for delivering the drug in the adhesive into the body through the skin. Hence there is a demand for selecting an adhesive base that minimizes such an interaction between the drug and the adhesive base.

In an example of the present invention, patch preparations containing rivastigmine as an active ingredient were prepared using various adhesive bases including acrylate, synthetic rubber, or natural rubber and evaluated in regard to 24-hour cumulative permeation amount ($\mu g/cm^2$), cumulative permeation amount (%) with respect to the initial content, and skin permeation rate ($\mu g/cm^2/h$), showing that the preparation using a styrene-isoprene-styrene block copolymer as a base was far superior in percutaneous permeation efficiency to the comparative preparations and the preparations commercially available.

Accordingly, the use of a styrene-isoprene-styrene block copolymer as an adhesive base makes it possible to transdermally deliver a necessary amount of drug enough for treatment of dementia using a smaller amount of rivastigmine than the conventional rivastigmine patch preparations, and to reduce skin irritation using a reduced amount of the drug.

The present invention is also characterized by using no more than a backing layer and a drug-containing layer without an additional intermediate layer or an additional adhesive layer.

As for the prior art, WO 99/34782 or WO 07/064407, using the drug-containing layer alone cannot provide properties sufficient to adhere the preparation to the skin and hence has an additional adhesive layer not affecting drug delivery into the skin, causing inconvenience of forming a separate layer and increasing the production cost. In contrast, the present invention including a styrene-isoprene-styrene block copolymer and a tackifier resin in the drug-containing layer provides good adhesion and wearing properties to the skin with a single adhesive layer containing the drug and the adhesive, thereby simplifying the manufacturing process of the prior art.

Preferably, the percutaneous absorption preparation of the present invention does not include a drug release controlling agent. The percutaneous absorption preparation of the present invention does not include a drug release controlling agent such as an organic acid or its salt; preferably the percutaneous absorption preparation does not include a drug release controlling agent such as an inorganic acid or its salt. Especially, the present invention does not additionally include acetate.

The prior art, EP 1437130 specifies that the use of an organic acid or its salt, particularly acetic acid, may enhance skin permeation of the drug and shows that without acetic acid, the skin permeation rate of the drug is considerably decreased to 0.1 to 0.4 $\mu g/cm^2/h$. As such, the prior art uses an organic acid salt, particularly acetate, as an essential ingredient for enhancing the skin permeability of the rivastigmine percutaneous preparation.

The present invention, however, uses a styrene-isoprene-styrene block copolymer as an adhesive base to acquire, relative to the conventional preparations, a high skin permeation rate (greater than 18 $\mu g/cm^2/h$) and a high cumulative skin permeation amount of rivastigmine (e.g., a 24-hour cumulative permeation amount is more than 30 % of the initial content) without incorporating an additional substance such as an organic acid salt or an inorganic acid salt.

The backing layer (a) of the percutaneous absorption preparation according to the present invention may use any drug-protecting substance used in the conventional percutaneous absorption preparations, with proviso that it is easy to adhere to the skin, prevents a loss of the drug pertaining to drug stability during storage, and has no reactivity to the skin, thus not causing allergies.

More specifically, the backing layer may be a laminate film including, but not specifically limited to, polyethylene, polypropylene, polyvinylacetate, polychlorovinylidene, ethylene vinylacetate copolymer, vinylon, polyester, polyurethane, nylon, polyacrylonitrile, polyethyleneterephthalate, polyolefin film, rayon, non-woven fabric, acryl, silk, cotton, aluminum sheet, or at two or more thereof.

The drug-containing layer (b) of the percutaneous absorption preparation according to the present invention

comprises, as an active ingredient, rivastigmine free base or its pharmaceutically acceptable salt. Rivastigmine is preferably rivastigmine free base, or rivastigmine hydrogen tartrate.

Due to using a styrene-isoprene-styrene block copolymer as an adhesive base, the percutaneous absorption preparation of the present invention can acquire a high skin permeability of the drug and hence use rivastigmine less than 1.8 mg/cm$^2$, which is the rivastigmine content of the conventional rivastigmine-containing percutaneous absorption preparations known in the prior art or commercially available, thereby reducing side effects such as skin irritation. The content of the rivastigmine free base or its pharmaceutically acceptable salt in the percutaneous absorption preparation of the present invention is at least 1.0 mg/cm$^2$ and less than 1.8 mg/ cm$^2$. The drug content less than 1.0 mg/cm$^2$ causes a deterioration of the drug permeation into the skin, hardly acquiring an effective potency. The drug content of 1.8 mg/cm$^2$ or more deteriorates of the cohesiveness of a polymer adhesive used as a base of the matrix layer due to the drug and causes skin irritation as a side effect of the drug.

The drug-containing layer of the percutaneous absorption preparation according to the present invention uses a styrene-isoprene-styrene block copolymer as a base. The content of the styrene-isoprene-styrene block copolymer in the matrix layer is preferably 20 to 50 wt.% with respect to the total weight of the drug-containing layer to satisfy a high permeation and to maintain a high cohesiveness for patch preparations.

The tackifier resin according to the present invention may include typical adhesives alone or in combination, such as, for example, natural resins, synthetic resins, or a mixture thereof. More specifically, the tackifier resin may include, but is not specifically limited to, at least one selected from the group consisting of alicyclic hydrocarbon resins such as a styrene alpha-methyl styrene resin (e.g., Kristalex F85); aliphatic hydrocarbon resins (e.g., Escorez 1401); hydrogenated hydrocarbon resins (e.g., Regalite R1100); rosin-based resins, such as rosin, hydrogenated rosin (e.g., KE311), or their esters (e.g., glycerol esters of rosin, or glycerin esters of hydrogenated rosin), or rosin derivatives such as pentaerythritol esters of rosin; terpene-based resins; and polyester resins such as maleic acid resin. Preferably, the tackifier may be one of alicyclic hydrocarbon resins or aliphatic hydrocarbon resins, or a mixture thereof.

The plasticizer may include, but is not specifically limited to, petroleum oils (e.g., paraffin process oil, naphthenic process oil, aromatic process oil, etc.), squalane, squalene, plant oils (e.g., olive oil, camellia oil, castor oil, tall oil, peanut oil, etc.), synthetic oils (e.g., silicone oil), dibasic acid esters (e.g., dibutyl phthalate, dioctyl phthalate, etc.), liquid rubber (e.g., polybutene, liquid isoprene rubber, etc.), liquid fatty acid esters (e.g., isopropyl myristate, hexyl laurate, diethyl sebacate, diisopropyl sebacate, etc.), diethylene glycol, polyethylene glycol, glycol salicylate, propylene glycol, dipropylene glycol, triacetin, triethyl citrate, and crotamiton, which are used alone or in combination.

Adhesiveness and cohesiveness high enough for patch preparation are taken into consideration in determining the content of the tackifier resin or the plasticizer. Preferably, the present invention uses, with respect to the total weight of the drug-containing matrix, 20 to 50 wt.% of the tackifier resin, and 1 to 20 wt.% of the plasticizer.

The drug-containing matrix layer (b) comprises the rivastigmine free base or its pharmaceutically acceptable salt, the styrene-isoprene-styrene block copolymer, the tackifier resin, and the plasticizer at a weight ratio of 1 ~ 2 : 2 ~ 5 : 2~5 : 0.1~2.

The percutaneous absorption preparation of the present invention can achieve the desired effect of the present invention with such a composition of the drug-containing layer that includes the rivastigmine free base or its pharmaceutically acceptable salt, the styrene-isoprene-styrene block copolymer, the tackifier resin, and the plasticizer.

More specifically, without using any other additives than the rivastigmine free base or its pharmaceutically acceptable salt, the styrene-isoprene-styrene block copolymer, the tackifier resin, and the plasticizer, the percutaneous absorption preparation of the present invention can achieve high potency with a small amount of rivastigmine less than 1.8 mg/cm$^2$, which is the rivastigmine content of the preparations commercially available, at least 1.0 mg/cm$^2$ and less than 1.8 mg/cm$^2$, preferably 1.0 to 1.5 mg/cm$^2$ (for example, 1.2 mg/cm$^2$); a daily drug release amount of 2 to 10 mg; a 24-hour cumulative permeation amount of the drug being 30 wt.% or more of the initial content (for example, 30 to 60 wt.% of the initial content); and a skin permeation rate of the drug greater than 18 $\mu$g/cm$^2$/h, particularly at least 20 $\mu$g/cm$^2$/h, more particularly 20 to 30 $\mu$g/cm$^2$/h.

When needed, the drug-containing layer of the present invention may further comprise at least one selected from the group consisting of a skin permeation enhancer, a UV blocking agent, and an antioxidant, which are optionally used in the conventional percutaneous absorption preparations for the purpose of enhancing compatibility with the drug and other excipients and stability of the preparation. But the addition of the skin permeation enhancer, the UV blocking agent, or the antioxidant is not necessary in the percutaneous absorption preparation of the present invention, which may achieve high potency and high skin permeability without using those additives.

The skin permeation enhancer is preferably used in an amount of 0.5 to 5 wt.% with respect to the total weight of the drug-containing layer and may include, but is not specifically limited to, fatty acids, fatty acid alcohols, patty acid esters, ionic or nonionic surfactants, isopropyl myristate, transcutol, triacetin, pyrrolidone compounds, etc.

The UV blocking agent may be a liquid organic UV blocking agent that is effective in UV absorption. The specific examples of the UV blocking agent include, but are not specifically limited to, ethylhexylmethoxy cinnamate, isoamyl P-methoxy cinnamate, bis-ethylhexyloxyphenol methoxyphenyl triazine, C12-15 alkyl benzoate, titanium oxide, aluminum

stearate, polyhydroxystearic acid, alumina, ethylhexyl salisilate, etc. Other organic UV absorbing agents that are effective in UV absorption and useful in the present invention may include, but are not specifically limited to, oxybenzone, octocrylene, diethylhexyl butamido triazone, 4-methylbenzylidene camphor, 3-benzylidene camphor, octyldimethyl PABA, anisotriazine, polysilicon, etc. The UV blocking agent used in the present invention may also be an inorganic UV blocking agent such as a solid powder UV filtering agent. The specific examples of the inorganic UV blocking agent include titanium dioxide, or zinc oxide. The content of the UV blocking agent may be controlled in consideration of the intended UV blocking effect.

The antioxidant is an antioxidizing compound or its derivatives or salts and may include, but is not specifically limited to, at least one selected from the group consisting of, for example, phenol-based antioxidants (e.g., tetrakis (3-(3,5-di-tert-butyl-4-hydroxyphenyl)propionate), triethylene glycol bis (3-tert-butyl-4-hydroxy-5-methylphenyl)propionate, bis-(2,4-di-tert-butyl-phenyl phosphate), octadecyl 3,5-di-(tert)-butyl-4-hydroxyhydrocinnamate, 2,4-bis (dodecylthiomethyl)-6-methylphenol, etc.), ascorbyl stearate, alpha lipoic acid, glutathione, coenzyme Q10, gamma linolenic acid, and linoleic acid.

The percutaneous absorption preparation for treatment of dementia according to the present invention may further comprise (c) a release liner. The release layer may be a release film or its laminate, which is typically used in the conventional percutaneous absorption preparations. The specific examples of the release layer include, but are not specifically limited to, polyethylene, polyester, polyvinyl chloride, polyvinylidene chloride films or papers coated with silicone resin or fluorocarbon resin coatings, or their laminates.

The percutaneous absorption preparation of the present invention is characterized by the *in vitro* 24-hour cumulative permeation amount of rivastigmine free base or its pharmaceutically acceptable salt that is 30 wt.% or more of the initial content, for example, 30 to 60 wt. % (as determined by the test described in Experimental Example 1).

The percutaneous absorption preparation can also be characterized by the daily release amount of rivastigmine free base or its pharmaceutically acceptable salt that is 2 to 10 mg as rivastigmine.

The percutaneous absorption preparation of the present invention is also characterized by the *in vitro* skin permeation rate of rivastigmine free base or its pharmaceutically acceptable salt that is greater than 18 $\mu g/cm^2/h$, for example, 20 $\mu g/cm^2/h$ or greater (e.g., 20 to 30 $\mu g/cm^2/h$) %, as determined by the test described in Experimental Example 1.

The present invention also provides a percutaneous absorption preparation comprising, as an active ingredient, rivastigmine free base or its pharmaceutically acceptable salt in an amount of at least 1.0 $mg/cm^2$ and less than 1.8 $mg/cm^2$, for example, 1.0 to 1.5 $mg/cm^2$ (e.g., 1.2 $mg/cm^2$), and having a daily drug release amount of 2 to 10 mg.

The present invention also provides a percutaneous absorption preparation comprising, as an active ingredient, rivastigmine free base or its pharmaceutically acceptable salt in an amount of at least 1.0 $mg/cm^2$ and less than 1.8 $mg/cm^2$, for example, 1.0 to 1.5 $mg/cm^2$, or 1.2 $mg/cm^2$, and having an *in vitro* skin permeation rate of the drug being greater than 18 $\mu g/cm^2/h$, for example, 20 $\mu g/cm^2/h$ or greater (e.g., 20 to 30 $\mu g/cm^2/h$).

In contrast to the conventional preparations that are problematic in regard to skin irritation, the percutaneous absorption preparation of the present invention causes little skin irritation and preferably has a primary irritation index (PII) of 0.3 or less, more preferably 0.25 or less, most preferably 0.2 or less as determined according to the Draize scoring criteria (1959).

The percutaneous absorption preparation for treatment of dementia containing rivastigmine as an active ingredient according to the present invention is effective for dementia treatment using a small amount of the active ingredient relative to the conventional preparations to reduce skin irritation and transdermally delivering a sufficient amount of drug for achieving a desired efficacy.

## BRIEF DESCRIPTION OF THE DRAWINGS

FIG. 1 is a cross-sectional diagram of the percutaneous absorption preparation for treatment of dementia containing rivastigmine as an active ingredient according to the present invention (A: backing layer; B: drug-containing layer; and C: release liner).

FIG. 2 is a graph showing the cumulative skin permeation amount of the percutaneous absorption preparation for treatment of dementia containing rivastigmine as an active ingredient according to the present invention.

## DETAILED DESCRIPTION OF THE PREFERRED EMBODIMENT

Hereinafter, the present invention will be described in detail with reference to examples and experimental examples, which are given only for illustrative purposes and not intended to limit the scope of the present invention.

Examples 1 to 6: Percutaneous Absorption Preparation for Treatment of Dementia Containing Rivastigmine as Active Ingredient

In a solvent, n-hexane, were dissolved a styrene-isoprene-styrene block copolymer (hereinafter, referred to as "SIS") as an adhesive base, polybutene (PB2400, Daelim Petrochemical Division) as a plasticizer, an alicyclic hydrocarbon resin (Kristalex F85, Eastman Chemical Company), aliphatic hydrocarbon resin (Escorez 1401, Exxonmobil Chemical), and rosin ester resin (KE311, Arakawa Chemical) to prepare an adhesive solution.

To the adhesive solution were added rivastigmine and optionally a skin permeation enhancer at a weight ratio of Table 1. The resultant homogeneous mixture was coated onto a siliconized polyester release film, which was then dried out at 90 °C for 5 minutes to prepare a sheet having a thickness as specified in Table 1. The sheet was laminated with a polyester backing film and cut in a defined size to obtain a percutaneous absorption preparation for treatment of dementia containing rivastigmine as an active ingredient.

[Table 1]

| Ingredient | | Example | | | | | | |
|---|---|---|---|---|---|---|---|---|
| | | 1 | 2 | 3 | 4 | 5 | 6 | 7 |
| Drug | Rivastigmine | 14 | 13 | 14 | 10 | 10 | 20 | 14 |
| Adhesive base | SIS | 39 | 39 | 39 | 38 | 38 | 42 | 38 |
| Tackifier resin | Kristalex F85 | 36 | 18 | 18 | | 20 | 36 | |
| | Escorez 1401 | | 18 | | 41 | 20 | | 36 |
| | KE311 | | | 18 | | | | |
| Plasticizer | Polybutene | 11 | 11 | 11 | 11 | 10 | 2 | 12 |
| Skin Permeation Enhancer | Laurylpyrrolidone | | | | | 1 | | |
| Thickness of Drug Layer (μm) | | 85 | 92 | 85 | 120 | 120 | 60 | 114 |
| Drug Content (mg/cm$^2$) | | 1.2 | 1.2 | 1.2 | 1.2 | 1.2 | 1.2 | 1.6 |

Comparative Examples 1 and 2

A rivastigmine-containing percutaneous absorption preparation was prepared in the same manner as described in Example 1, excepting that the adhesive base of the drug-containing matrix layer and the ingredients given as shown in Table 1 were used. A rivastigmine patch (Exelon, drug content 18 mg, 10 cm$^2$) commercially available was used as Comparative Example 3. The compatibility with the drug was poor when using polyisobutene as a base in Comparative Example 1.

[Table 2]

| Ingredient | | Comparative Example | |
|---|---|---|---|
| | | 1 | 2 |
| Drug | Rivastigmine | 14 | 20 |
| Adhesive base | Polyisobutylene (PIB) | 39 | |
| | Duro-Tak 87-4098 (Acrylic adhesive having no functional group) | | 80 |
| Tackifier resin | Kristalex F85 | 36 | |
| Plasticizer | Polybutene | 11 | |
| Thickness of Drug Layer (μm) | | 85 | 60 |
| Drug Content (mg/cm$^2$) | | 1.2 | 1.2 |

Experimental Example 1: Skin Permeation Test

A Franz diffusion cell system was used as an apparatus for transdermal permeation test, which used a phosphate buffer solution (pH 7.4) containing 0.5 % sodium azide that was kept at 32 °C and stirred at 600 rpm during the test. A circular piece (0.636 $cm^2$) was cut out from each of the percutaneous absorption preparation of Examples and Comparative Examples and applied on human cadaver skin epidermis, which was then placed on the Franz diffusion cell system. 100 $\mu$l of the sample was collected and analyzed using HPLC to determine a 24-hour cumulative permeation amount ($\mu g/cm^2$), a cumulative permeation amount (%) with respect to the initial content, and a skin permeation rate ($\mu g/cm^2/h$) for the percutaneous absorption preparation containing rivastigmine. The results are presented in Table 3.

[Table 3]

| | Rivastigmine Cumulative Permeation Amount ($\mu g/cm^2$) | Cumulative Permeation Amount (%) with respect to Initial Content | Rivastigmine Skin Permeation Rate ($\mu g/cm^2/h$) |
|---|---|---|---|
| Example 1 | 570.7 ± 44.9 | 47.56 | 23.75 |
| Example 2 | 438.6 ± 27.3 | 36.5 | 18.27 |
| Example 3 | 440.6 ± 32.2 | 36.72 | 18.36 |
| Example 4 | 455.2 ± 32.5 | 37.90 | 18.97 |
| Example 5 | 499.7 ± 12.8 | 41.64 | 20.82 |
| Example 6 | 703.5 ± 26.1 | 43.9 | 29.31 |
| Example 7 | 583.2 ± 36.4 | 32.40 | 24.30 |
| Comparative Example 1 | 252.0 ± 15.6 | 21.00 | 10.50 |
| Comparative Example 2 | 229.7 ± 19.5 | 19.14 | 9.57 |
| Comparative Example 3 | 437.1 ± 26.7 | 24.28 | 18.21 |

As can be seen from Table 3, the percutaneous absorption preparations using an SIS adhesive other than an acrylic adhesive or a polyisobutylene adhesive showed a skin permeation amount of the drug equivalent to or higher than that of the conventional products, achieving an effective rivastigmine delivery for treatment of dementia.

Experimental Example 2: Skin Irritation Test

Each of the percutaneous absorption preparations of Examples and Comparative Examples was placed on the shaved skin of a test rabbit and observed for signs of skin irritation such as erythema, eschar, or edema for 12 or 24 hours. Skin reaction were scored at time intervals according to the evaluation method (Draize skin reaction evaluation table (Draize criteria: 1959)), and the sum of the time-based skin reaction scores was divided by the number of objects to determine the average score. The sum of the average scores was divided by 4 to determine a primary irritation index (PII) for skin irritation grading.

[Table 4]

| Draize Skin Reaction Assay (Draize Criteria: 1959) | |
|---|---|
| Reaction | Average Score |
| **Erythema / Eschar** | |
| No erythema | 0 |
| Very slight erythema, barely perceptible | 1 |
| Well-defined erythema | 2 |
| Moderate to severe erythema | 3 |

(continued)

| Draize Skin Reaction Assay (Draize Criteria: 1959) | |
|---|---|
| Reaction | Average Score |
| **Erythema / Eschar** | |
| Severe erythema (beet redness) to slight eschar formation | 4 |
| | Highest Score = 4 |
| **Edema** | |
| No edema | 0 |
| Very slight edema, barely perceptible | 1 |
| Slight edma (edges of area well defined by raising) | 2 |
| Moderate edema (raised approximately 1 mm or more) | 3 |
| Severe edema (raised more than 1 mm and extending beyond the area of exposure) | 4 |
| | Highest Score = 4 |

Average score = the sum of skin reaction scores with the lapse of time / the number of objects

$$\text{PII value} = \text{the sum of average scores} / 4$$

[Table 5]

| Primary Skin Irritation Index (PII) | |
|---|---|
| Primary skin irritation index PII) | Div. |
| 0 | Non-irritating |
| 0 < PII value ≤ 2 | Slightly irritating |
| 2 < PII value ≤ 5 | Moderately irritating |
| 8 < PII value ≤ 8 | Severely irritating |

[Table 6]

| Preparation | Primary Skin Irritation Index (PII) |
|---|---|
| Example 1 | 0 |
| Example 6 | 0.25 |
| Comparative Example 3 | 0.35 |

The evaluation demonstrated that the preparation of the present invention reduced skin irritation relative to the preparation commercially available.

Experimental Experiment 3. Measurement of residual drug amounts after application to the skin

The percutaneous absorption patch preparations prepared according to the examples and the comparative examples were cut and applied to the skin of the humeral region for 24 hours. Then, the patches were removed from the skin to measure residual drug amounts in the patch, thereby to derive drug amounts released from the patches. The results are shown in the following Table 7.

[Table 7]

| Preparation | Total drug in the patch(mg) | Residual drug in the patch(mg) | Released drug (mg) |
|---|---|---|---|
| Example 1 | 6mg/5cm$^2$ | 2.54±0.67 | 3.46±0.67 |
| Example 2 | 6mg/5cm$^2$ | 3.08±0.73 | 2.92±0.73 |
| Example 6 | 12mg/10cm$^2$ | 4.09±0.73 | 7.91±1.47 |
| Comparative Example 3 | 9mg/5cm$^2$ | 6.13±0.42 | 2.87±0.42 |

## Claims

1. A percutaneous absorption preparation comprising:

   (a) a backing layer; and
   (b) a drug-containing layer comprising rivastigmine free base or a pharmaceutically acceptable salt thereof, a styrene-isoprene-styrene block copolymer, a tackifier resin, and a plasticizer

   wherein the percutaneous absorption preparation is free from an additional intermediate layer or an additional adhesive layer;
   the drug-containing layer (b) does not comprise an organic acid or a salt thereof; and the rivastigmine free base or pharmaceutically acceptable salt thereof is included in an amount of at least 1.0 mg/cm$^2$ and less than 1.8 mg/cm$^2$, and the rivastigmine free base or pharmaceutically acceptable salt thereof, the styrene-isoprene-styrene block copolymer, the tackifier resin, and the plasticizer are included at a weight ratio of 1~2 : 2~5 : 2~5 : 0.1~2.

2. The percutaneous absorption preparation as claimed in claim 1, wherein the drug-containing layer (b) does not comprise an antioxidant.

3. The percutaneous absorption preparation as claimed in claim 1, wherein the tackifier resin is a natural resin, a synthetic resin, or a mixture thereof.

4. The percutaneous absorption preparation as claimed in claim 3, wherein the tackifier resin is at least one selected from the group consisting of an alicyclic hydrocarbon resin, an aliphatic hydrocarbon resin, a hydrogenated hydrocarbon resin, a rosin-based resin, a terpene-based resin, and a polyester resin.

5. The percutaneous absorption preparation as claimed in claim 1, wherein the plasticizer is at least one selected from the group consisting of petroleum oil, squalane, squalene, plant oil, synthetic oil, dibasic acid ester, liquid rubber, liquid fatty acid ester, diethylene glycol, polyethylene glycol, glycol salicylate, propylene glycol, dipropylene glycol, triacetin, triethyl citrate, and crotamiton.

6. The percutaneous absorption preparation as claimed in claim 1, wherein the rivastigmine free base or pharmaceutically acceptable salt thereof has an *in vitro* 24-hour cumulative permeation amount of 30 wt.% or more with respect to the initial content thereof, as determined in a transdermal permeation test on skin epidermis from human cadaver using a Franz diffusion cell system containing a phosphate buffer solution at pH 7.4 containing 0.5 % sodium azide kept at 32 °C and stirred at 600 rpm during the test.

7. The percutaneous absorption preparation as claimed in claim 1, wherein the rivastigmine free base or pharmaceutically acceptable salt thereof has an *in vitro* skin permeation rate greater than 18 μg/cm$^2$/h, as determined in a transdermal permeation test on skin epidermis from human cadaver using a Franz diffusion cell system containing a phosphate buffer solution at pH 7.4 containing 0.5 % sodium azide kept at 32 °C and stirred at 600 rpm during the test.

8. The percutaneous absorption preparation as claimed in any one of claims 1 to 7, further comprising:
   (c) a release liner.

## Patentansprüche

1. **1.** Perkutane Absorptionsherstellung, umfassend:

    (a) eine Rückschicht; und
    (b) eine arzneimittelhaltige Schicht, umfassend Rivastigmin freie Base oder ein pharmazeutisch verträgliches Salz davon, ein Styrol-Isopren-Styrol-Blockcopolymer, ein Klebrigmacherharz und einen Weichmacher,

    wobei die perkutane Absorptionsherstellung frei von einer zusätzlichen Zwischenschicht oder einer zusätzlichen Klebeschicht ist,
    die arzneimittelhaltige Schicht (b) keine organische Säure oder ein Salz davon umfasst; und die Rivastigmin freie Base oder das pharmazeutisch verträgliche Salz davon in einer Menge von mindestens 1,0 mg/cm$^2$ und weniger als 1,8 mg/cm$^2$ enthalten ist, und
    die Rivastigmin freie Base oder das pharmazeutisch verträgliche Salz davon, das Styrol-Isopren-Styrol-Blockcopolymer, das Klebrigmacherharz und der Weichmacher in einem Gewichtsverhältnis von 1 ~ 2 : 2 ~ 5 : 2 ~ 5 : 0,1 ~ 2 enthalten sind.

**2.** Perkutane Absorptionsherstellung wie in Anspruch 1 beansprucht, wobei die arzneimittelhaltige Schicht (b) kein Antioxidans umfasst.

**3.** Perkutane Absorptionsherstellung wie in Anspruch 1 beansprucht, wobei es sich bei dem Klebrigmacherharz um ein natürliches Harz, ein synthetisches Harz oder ein Gemisch davon handelt.

**4.** Perkutane Absorptionsherstellung wie in Anspruch 3 beansprucht, wobei es sich bei dem Klebrigmacherharz um mindestens eines handelt, ausgewählt aus der Gruppe, bestehend aus einem alizyklischen Kohlenwasserstoffharz, einem aliphatischen Kohlenwasserstoffharz, einem hydrogenierten Kohlenwasserstoffharz, einem Rosinbasierten Harz, einem Terpen-basierten Harz und einem Polyesterharz.

**5.** Perkutane Absorptionsherstellung wie in Anspruch 1 beansprucht, wobei es sich bei dem Weichmacher um mindestens einen handelt, ausgewählt aus der Gruppe, bestehend aus Mineralöl, Squalan, Squalen, Pflanzenöl, synthetischem Öl, dibasischem Säureester, Flüssiggummi, flüssigem Fettsäureester, Diethylenglykol, Polyethylenglykol, Glykolsalicylat, Propylenglykol, Dipropylenglykol, Triacetin, Triethylcitrat und Crotamiton.

**6.** Perkutane Absorptionsherstellung wie in Anspruch 1 beansprucht, wobei die Rivastigmin freie Base oder das pharmazeutisch verträgliche Salz davon eine kumulative 24-Std.-*in-vitro*-Permeationsmenge von 30 Gew.-% oder mehr in Bezug auf deren Anfangsgehalt aufweist, bestimmt in einem transdermalen Permeationstest auf Haut-Epidermis von einem menschlichen Leichnam unter Verwendung eines Franz-Diffusionszell-Systems, welches eine 0,5 % Natriumazid enthaltende Phosphatpufferlösung bei pH 7,4 enthält, die während des Tests bei 32 °C gehalten und bei 600 rpm gerührt wird.

**7.** Perkutane Absorptionsherstellung wie in Anspruch 1 beansprucht, wobei die Rivastigmin freie Base oder das pharmazeutisch verträgliche Salz davon eine *in-vitro*-Hautpermeationsgeschwindigkeit von größer als 18 μg/cm$^2$/h aufweist, bestimmt in einem transdermalen Permeationstest auf Haut-Epidermis von einem menschlichen Leichnam unter Verwendung eines Franz-Diffusionszell-Systems, welches eine 0,5 % Natriumazid enthaltende Phosphatpufferlösung bei pH 7,4 enthält, die während des Tests bei 32 °C gehalten und bei 600 rpm gerührt wird.

**8.** Perkutane Absorptionsherstellung wie in einem der Ansprüche 1 bis 7 beansprucht, ferner umfassend:
(c) eine Trennlage.

## Revendications

**1.** Préparation pour absorption percutanée comprenant :

(a) une couche de support ; et
(b) une couche contenant un médicament, comprenant de la rivastigmine sous forme de base libre ou d'un sel pharmaceutiquement acceptable de celle-ci, un copolymère séquencé de styrène-isoprène-styrène, une résine donnant du collant, et un plastifiant,

laquelle préparation pour absorption percutanée est exempte de couche intermédiaire additionnelle ou de couche adhésive additionnelle ; dans laquelle
la couche contenant un médicament (b) ne comprend pas un acide organique ou un sel de celui-ci ; et la rivastigmine sous forme de base libre ou d'un sel pharmaceutiquement acceptable de celle-ci est incluse en une quantité d'au moins 1,0 mg/cm$^2$ et inférieure à 1,8 mg/cm$^2$, et
la rivastigmine sous forme de base libre ou d'un sel pharmaceutiquement acceptable de celle-ci, le copolymère séquencé de styrène-isoprène-styrène, la résine donnant du collant, et le plastifiant sont inclus en un rapport en poids de 1~2/2~5/2~5/0,1~2.

**2.** Préparation pour absorption percutanée selon la revendication 1, dans laquelle la couche contenant un médicament (b) ne comprend pas d'antioxydant.

**3.** Préparation pour absorption percutanée selon la revendication 1, dans laquelle la résine donnant du collant est une résine naturelle, une résine synthétique, ou un mélange de celles-ci.

**4.** Préparation pour absorption percutanée selon la revendication 3, dans laquelle la résine donnant du collant est au moins l'une choisie dans le groupe constitué par une résine hydrocarbonée alicyclique, une résine hydrocarbonée aliphatique, une résine hydrocarbonée hydrogénée, une résine à base de colophane, une résine à base de terpène, et une résine de polyester.

**5.** Préparation pour absorption percutanée selon la revendication 1, dans laquelle le plastifiant est au moins l'un choisi dans le groupe constitué par l'huile de pétrole, le squalane, le squalène, une huile végétale, une huile synthétique, un ester d'acide dibasique, un caoutchouc liquide, un ester d'acide gras liquide, le diéthylèneglycol, le polyéthylèneglycol, le salicylate de glycol, le propylèneglycol, le dipropylèneglycol, la triacétine, le citrate de triéthyle, et le crotamiton.

**6.** Préparation pour absorption percutanée selon la revendication 1, dans laquelle la rivastigmine sous forme de base libre ou d'un sel pharmaceutiquement acceptable de celle-ci a une quantité de perméation cumulée sur 24 heures *in vitro* de 30% en poids ou plus par rapport à sa teneur initiale, telle que déterminée dans un test de perméation transdermique sur un épiderme de peau provenant d'un cadavre humain utilisant un système de cellule de diffusion de Franz contenant une solution tampon phosphate à pH 7,4 contenant 0,5% d'azoture de sodium maintenue à 32°C et agitée à 600 t/min durant le test.

**7.** Préparation pour absorption percutanée selon la revendication 1, dans laquelle la rivastigmine sous forme de base libre ou d'un sel pharmaceutiquement acceptable de celle-ci a une vitesse de perméation à travers la peau *in vitro* supérieure à 18 $\mu$g/cm$^2$/h, telle que déterminée dans un test de perméation transdermique sur un épiderme de peau provenant d'un cadavre humain utilisant un système de cellule de diffusion de Franz contenant une solution tampon phosphate à pH 7,4 contenant 0,5% d'azoture de sodium maintenue à 32°C et agitée à 600 t/min durant le test.

**8.** Préparation pour absorption percutanée selon l'une quelconque des revendications 1 à 7, comprenant en outre :
(c) une protection anti-adhésive.

[FIG. 1]

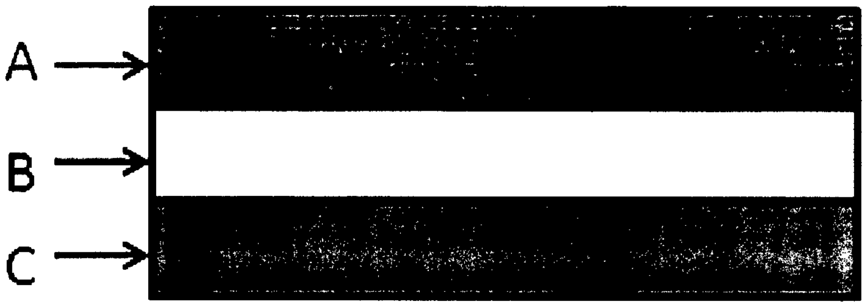
A
B
C

[FIG. 2]

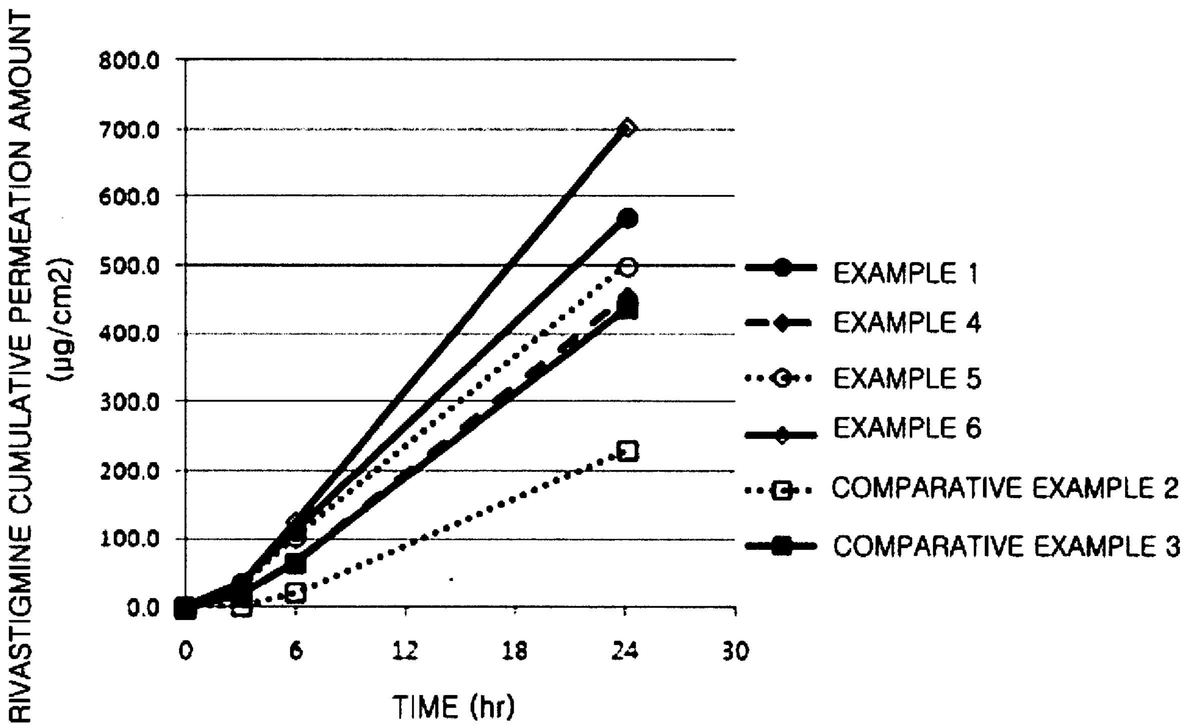
RIVASTIGMINE CUMULATIVE PERMEATION AMOUNT (μg/cm2)
800.0
700.0
600.0
500.0
400.0
300.0
200.0
100.0
0.0
0
6
12
18
24
30
TIME (hr)
EXAMPLE 1
EXAMPLE 4
EXAMPLE 5
EXAMPLE 6
COMPARATIVE EXAMPLE 2
COMPARATIVE EXAMPLE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9934782 A
- WO 07064407 A
- EP 1437130 A
- JP 2009022730 A


### Non-patent literature cited in the description


- *Clinical Drug Investigation,* 01 January 2010, vol. 30, 41-49